# EUROPEAN PATENT APPLICATION

(11) **EP 2 813 527 A1**
(43) Date of publication of application: **17.12.2014**
(21) Application number: 13746207.3
(22) Date of filing: 04.02.2013
(51) Int. Cl.: C08G 63/85, C08G 63/12, C08L 67/02, A61F 13/49

(54) **CATALYST FOR SYNTHESIS OF POLYESTER RESIN AND METHOD FOR PRODUCING POLYESTER RESIN USING SAME**

(30) Priority: 06.02.2012 KR 20120011920
(71) Applicant: Samsung Fine Chemicals Co., Ltd., Ulsan 680-090 (KR); S-Enpol Co., Ltd., Wonju-si, Gangwon-do 220-805 (KR)
(72) Inventor: KANG, Gyung Don, Daejeon 305-768 (KR); YOON, Ki Chul, Cheonan-si Chungcheongnam-do 330-260 (KR); AHN, Ji Soo, Daejeon 305-744 (KR); LEE, Chung Il, Seoul 137-060 (KR); KIM, Ye Jin, Seoul 130-100 (KR); PARK, Sung Bae, Incheon 407-788 (KR); KIM, Se Hoon, Wonju-si Gangwon-do 220-805 (KR)
(74) Representative: Kador & Partner
(86) International application number: PCT/KR2013/000876
(87) International publication number: WO 2013/119004

(57) **Abstract**

A catalyst used to manufacture a biodegradable polyester resin, in particular, an organic titanium catalyst which does not contain a heavy-metal component, and a method of manufacturing a polyester resin using the same. Accordingly, the method of manufacturing a polyester resin can be useful in attaining desired physical properties without using a catalyst harmful to environments and a human body. The biodegradable resin, which can be safely used in the field of applications in which the use of harmful components is a sensitive issue without using the components harmful to environments and a human body during the manufacture of a resin, can be prepared.

## Description

### [Technical Field]

The present invention relates to a catalyst used in a process of manufacturing a polyester resin which is a biodegradable polymer, and a method of manufacturing a polyester resin using the same. More particularly, the present invention relates to a method of manufacturing a polyester resin suitable to be used in the applications where the use of harmful components is a sensitive issue, using a catalyst containing environmentally friendly organic components instead of the components harmful to human bodies and environments.

### [Background Art]

A biodegradable resin stands for a synthetic resin developed as a new material which does not cause environmental pollution as it being decomposed to water and carbon dioxide and water and by naturally existing microorganisms such as bacteria, algae and fungi. Examples of the biodegradable resin include cellulose polymers, starches, and polylactides (PLA), and also include polyester resins such as poly(butylene succinate) (PBS), poly(butylene succinate-co-adipate) (PBSA) and poly(butylene adipate-co-terephthalate) (PBAT) to which many attentions are paid as having superior mechanical strength.

During the polymerization of a polyester resin, a catalyst is used in order to improve qualities or production efficiency of the resin. When a polyester resin is manufactured, a catalyst is a key material for qualities and production efficiency of the resin, and thus new catalysts have been continuously developed among the technologies for manufacturing a polyester resin.

A heavy metal catalyst having a superior catalytic effect as the polymerization catalyst for a polyester resin includes tin (Sn), antimony (Sb) and germanium (Ge). The metal catalyst has both of advantages and disadvantages in terms of performance and cost. Although an antimony-based catalyst is inexpensive, it causes environmental issues. Also, it may cause discoloration in the prepared polyester resin. On the other hands, a germanium-based catalyst may be used to obtain a resin having high transparency, but has a problem in that it is too expensive to be applied for mass production.

Because of the above-described disadvantages, there is a continuous demand for an inexpensive and environmentally friendly catalyst without any heavy metal despite keeping the performance of a catalyst intact. Considering the aspects of cost and efficiency, the most commercially successful catalyst is an antimony-based catalyst. As explained above, however, the antimony-based catalyst has undesirable influence on environment. Therefore, there is ardent research to replace the antimony-based catalyst.

Especially, the polyester resin to which many attentions are paid as a biodegradable resin is restricted by the use of a heavy-metal catalyst which is a harmful and poisonous material. For this reason, biodegradable polyester resin has been manufactured without any harmful material for applications in field of a diaper or food packaging or a medicine packaging materials.

### [Disclosure]

### [Technical Problem]

An object of the present invention is to provide a method of manufacturing a polyester resin by polymerization reaction of an alcohol monomer and a carboxylic acid monomer without using a heavy-metal catalyst which is harmful to a human body and environment, and, more particularly, to provide a polyester resin which can be safely used as a biodegradable resin in the field of applications in which the use of harmful components is a sensitive issue.

### [Technical Solution]

According to an aspect of the present invention, there is provided a method of manufacturing a polyester resin. Here, the method includes esterifying an alcohol monomer and a carboxylic acid monomer at a temperature of 200 to 220 °C in the presence of 0.1 to 1.5 g of an organic titanium catalyst with respect to 1 mol of the carboxylic acid monomer, and polycondensing the esterification reaction product at a temperature of 220 to 240 °C under a vacuum of less than 2 Torr.

In the esterification of the alcohol monomer and the carboxylic acid monomer, the alcohol monomer may be at least one selected from the group consisting of 1,4-butylene glycol, 1,3-butylene glycol, 1,3-propylene glycol and 1,2-ethylene glycol, and the carboxylic acid monomer may be at least one selected from the group consisting of succinic acid, adipic acid, suberic acid, sebacic acid, terephthalic acid, their anhydrides and their derivatives, in the step of esterification reaction.

The esterification may be performed using an organic titanium catalyst including 5 to 15 wt% of titanium.

Triphenyl phosphate or trimethyl phosphate may be further used in the esterification.

According to another aspect of the present invention, there is provided a polyester resin manufactured by the method.

According to still another aspect of the present invention, there is provided an article including the polyester resin.

The article may be a diaper, a food packaging material or a medicine packaging material.

According to yet another aspect of the present invention, there is provided an organic titanium catalyst including 5 to 15 wt% of a titanium component used for an esterification reaction of an alcohol monomer and a carboxylic acid monomer.

### [Advantageous Effects]

According to the present invention, a polyester resin, which does not contain a component which causes environmental pollution and is harmful to a human body, can be manufactured using a catalyst which does not contain a heavy-metal harmful to a human body and environments as a catalyst for polymerization reaction to manufacture a polyester resin.

Thus, the polyester resin provided by the present invention has advantages in that it can be used for an article, such as a diaper, which is brought into direct contact with a human body, or an article, such as a food packaging material and a medicine packaging material, in which the use of harmful materials is a sensitive issue.

### [Mode for Invention]

The present invention provides a catalyst for synthesizing a polyester resin by polymerization reaction of an alcohol monomer and a carboxylic acid monomer, for example, an environmentally friendly organic titanium catalyst which does not contain a heavy-metal component, and a method of manufacturing a polyester resin, which has physical properties suitable for use as a biodegradable resin, using the same.

In particular, according to the present invention, the polyester resin is manufactured by esterifying an alcohol monomer and a carboxylic acid monomer at a temperature of 200 to 220 °C in the presence of 0.1 to 1.5 g of an organic titanium catalyst with respect to 1 mol of the carboxylic acid monomer, and polycondensing the esterification reaction product at a temperature of 220 to 240 °C under a vacuum of less than 2 Torr. In other words, according to the present invention, the biodegradable resin which could attain desired physical properties may be prepared without any heavy-metal catalyst by controlling an amount of the organic titanium catalyst used and a reaction temperature in the esterification and polycondensation.

In the esterification reaction, at least one selected from the group consisting of 1,4-butylene glycol, 1,3-butylene glycol, 1,3-propylene glycol and 1,2-ethylene glycol may be used as the alcohol monomer, and at least one selected from the group consisting of succinic acid, adipic acid, suberic acid, sebacic acid, terephthalic acid, their anhydrides and their derivatives may be used as the carboxylic acid monomer.

The organic titanium catalyst including 5 to 15 wt% of a titanium component is used in the esterification reaction. Here, tetra-n-butyl titanate (Ti(OC₄H₉)₄) which contains a titanium component at a content of 14.0±0.1%, or Vertec VEXP 0641 (titanium type catalyst available from Johnson Matthey) which contains a titanium component at a content of 7.0% may be used as the organic titanium catalyst. The amount of the catalyst and the reaction conditions need to be controlled in order to manufacture a polyester resin using the organic titanium catalyst including a titanium component within this range.

The preferred amount of the catalyst used is in a range of 0.1 to 1.5 g, based on 1 mol of the carboxylic acid. When the polyester resin is prepared from the alcohol monomer and the carboxylic acid monomer, the alcohol monomer is added at an excessive amount, preferably at a content equivalent to 1 to 1.5, and, more preferably 1 to 1.2, based on the carboxylic acid monomer. This is because synthesizing oligomers containing hydroxyl end groups thereof during the esterification using an excessive amount of the alcohol monomer and then removing excess glycol for the alcoholysis reaction of the hydroxyl end groups during the polycondensation is easier than removing dicarboxylic acid for the acidolysis of oligomers containing carboxyl end groups thereof. As a result, an amount of the organic titanium catalyst used may be determined according to an amount of the carboxylic acid monomer.

When the organic titanium catalyst is used less than this range, the production efficiency may be reduced due to a decrease in reaction rate. On the other hands, when the organic titanium catalyst is used exceeding this range, yellowing may occur in the prepared resin.

Also, a catalyst including 5 to 15 wt% of a titanium component with respect to the total weight of the catalyst may be used as the preferred organic titanium catalyst which is used within this range. The amount of the titanium component included in the organic titanium catalyst varies depending on the catalyst. Accordingly, the amount of the catalyst may be controlled to manufacture a resin having optimal effects, that is, desirable physical properties, depending on the catalyst used for the reaction.

For example, as described above, tetra-n-butyl titanate including 14.0% of a titanium component may be used in a range of 0.25 to 0.5 g with respect to 1 mol of the carboxylic acid monomer to achieve the most preferable effects. On the other hands, when Vertec VEXP 0641 which contains a relatively small amount of a titanium component is used as the catalyst, the catalyst may be used in a range of 0.5 to 1.0 g in view of physical properties of the prepared resin.

After determination for contents of catalyst, the alcohol monomer and the carboxylic acid monomer are reacted at a temperature of 200 to 220 °C in the presence of the organic titanium catalyst in the esterification. An esterification reaction may not be easily performed at the temperature less than this temperature range. At the temperature exceeding this temperature range, the reaction may be swiftly performed but undesirable side reaction may occur.

Also, the esterification may proceed by further adding a phosphorous compound such as triphenyl phosphate or trimethyl phosphate. The phosphorous compound is used as a thermal decomposition stabilizer, which maintains a stable reaction by preventing thermal decomposition when a polymerization reaction is conducted at a high temperature.

The esterification proceeds until condensates such as water, methanol, etc. are discharged at a theoretically calculated amount. The reaction product formed by the esterification is an oligomer level.

Thereafter, the polycondensation reaction is performed at a temperature of 220 to 240 °C under a vacuum of less than 2 Torr. The polycondensation reaction is to perform polymerization between the oligomers formed in the esterification reaction, at an oligomer level or not having a desired molecular weight. In this case, the polycondensation reaction proceeds through an unreacted functional groups of an end of a polymer chain or an unreacted functional groups in a mid of polymer chain. Accordingly, the reaction is conducted at a higher temperature than that of the esterification under a vacuum condition. Similarly, the polycondensation reaction does not occur at a satisfactory level at a temperature lower than this range. Also, at a higher temperature exceeding this range, the reaction may be performed so fast that side effects such as a decrease in molecular weight by thermal decomposition and yellowing can occur.

The obtained biodegradable polyester resin has desirable physical properties suitable for various applications in aspects of its color and melting flow index.

According to the present invention, the polyester resin manufactured by the method is prepared without any component harmful to a human body and environments. And thus it can be safely used as a biodegradable resin, particularly, in a diaper which is brought into direct contact with a human body, or in the field of applications in which the use of harmful components is a sensitive issue, such as a food packaging or medicine packaging material.

Additionally, the present invention provides an organic titanium catalyst used as a catalyst for polymerization reaction to manufacture polyester resin. The organic titanium catalyst may include a titanium component at a content of 5 to 15 wt%, based on the total weight of the catalyst. The polyester resin having the physical properties suitable for use as a biodegradable resin may be prepared using the catalyst including a titanium component. A range of the catalyst content is 0.1 to 1.5 g based on 1 mol of the carboxylic acid monomer used in the polymerization of a polyester resin by controlling a reaction temperature for esterification of a monomer to 200 to 220 °C, followed by controlling a reaction temperature for polycondensation reaction to 220 to 240 °C.

The present invention will be explained in further detail with reference to Examples as described below. While the present invention is shown and described in connection with exemplary embodiments thereof, it will be apparent to those skilled in the art that various changes and modifications can be made without departing from the scope of the invention.

### Example 1

### [Esterification]

To a 500 ml 3-neck glass flask (Pyrex) equipped with a mechanical agitator and a condenser, 1 mol of succinic acid, 1.15 mol of 1,4-butylene glycol, 0.1 g of triphenyl phosphate as a thermal decomposition stabilizer, and tetra-n-butyl titanate as a catalyst at an amount described in Table 1 below were added. An esterification reaction was conducted at 220 °C for 2 hours at an agitation speed of 80 rpm. The reaction was terminated when a theoretical amount, i.e., 2mol (36g) of water generated by the reaction of monomers was discharged.

### [Polycondensation reaction]

A round-bottom flask in which the esterification reaction was terminated was moved to an oil bath whose initial temperature was set to 220 °C, and a vacuum pump was driven to gradually establish a vacuum of 2 Torr in the flask while agitating using a mechanical agitation device. Thereafter, a polycondensation reaction was performed at 240 °C for 3 hours, thus obtaining a final polyester resin.

### Examples 2 to 5

Polyester resins were prepared by the same method as in Example 1, except that the amount of the tetra-n-butyl titanate catalyst and the reaction temperature in the esterification were controlled as indicated in Table 1 below.

### Example 6

### [Esterification]

To a 500 ml 3-neck glass flask (Pyrex) equipped with a mechanical agitator and a condenser, 0.48 mol of dimethyl terephthalate, 1.3 mol of 1,4-butylene glycol, 0.1 g of triphenyl phosphate as a thermal decomposition stabilizer, and VEXP 0641 (available from Johnson Matthey) as a catalyst in an amount described in Table 1 below were added. An esterification reaction was conducted at 220 °C for 1 hour at an agitation speed of 80 rpm.

After a discharge process of methanol, 0.52 mol of adipic acid was added when the temperature at upper part of the flask started to decrease, and an esterification reaction was conducted for 1 hour under the same conditions by repeatedly performing the previous procedures.

### [Polycondensation reaction]

A round-bottom flask in which the esterification reaction was terminated was moved to an oil bath whose initial temperature was set to 220 °C, and a vacuum pump was driven to gradually establish a vacuum of 2 Torr in the flask while agitating using a mechanical agitation device. Thereafter, an excessive amount of glycol was removed at 240 °C, and a polycondensation reaction was then performed for 3 hours, thus obtaining a final polyester resin.

### Examples 7 to 10

Polyester resins were prepared by the same method as in Example 6, except that the amount of the Vertec VEXP 0641 catalyst and the reaction temperature in the esterification were controlled as indicated in Table 1 below.

### Comparative Example 1

### [Esterification]

An esterification reaction was conducted by the same method as in the esterification in Example 1, except that 0.11 g of antimony tri-oxide, 0.11 g of dibutyl tin oxide, and 0.4 g of tetra-n-butyl titanate were used as the catalysts and the reaction temperature was set to 180 °C.

### [Polycondensation reaction]

A polyester resin was prepared by the same polycondensation method as in the polycondensation in Example 1, except that the reaction temperature was set to 210 °C.

### Comparative Example 2

### [Esterification]

An esterification reaction was conducted by the same method as in the esterification in Example 6, except that 0.03 g of antimony tri-oxide and 0.2 g of tetra-n-butyl titanate were used as the catalysts and the reaction temperature was set to 180°C.

### [Polycondensation]

A polyester resin was prepared by the same polycondensation as in the polycondensation in Example 6, except that the reaction temperature was set to 210 °C.

### Test for physical properties of the polyester resin

The polyester resins prepared in Examples 1 to 10 and Comparative Examples 1 and 2 were tested for color and melting flow index (MFI). The colors of the resins was measured, using a colorimeter (SpectraMagic NX available from Konica Minolta), to determine color indexes (L, a and b values). For MFI, MELTINDEXER (GOTPFERT, MI-3) was used and the amount of flow at 190 °C under a load of 2,160 g for 10 minutes was measured. The results are summarized in Table 1 below.

**Table 1**

| | Sb or Sn (g) | Ti (g) | Vertec VEXP 0641 (g) | Esterification temperature (°C) | polycondensa tion temperature (°C) | Color (L, a, b) | MFI |
|---|---|---|---|---|---|---|---|
| Example 1 | | 0.3 | | 220 | 240 | 84.1/-1.5/4.2 | 6.9 |
| Example 2 | | 0.34 | | 210 | 230 | 85.6/-0.8/5.0 | 5.5 |
| Example 3 | | 0.4 | | 200 | 230 | 85.3/-1.1/5.2 | 4.8 |
| Example 4 | | 0.5 | | 200 | 240 | 83.6/-1.5/4.8 | 7.6 |
| Example 5 | | 0.6 | | 200 | 240 | 82.3/-0.5/8.2 | 7.3 |
| Example 6 | | | 0.4 | 220 | 240 | 87.3/-1.3/6.6 | 12.1 |
| Example 7 | | | 0.6 | 210 | 240 | 86.5/-1.7/5.4 | 8.1 |
| Example 8 | | | 0.8 | 200 | 230 | 85.7/-1.6/5.5 | 7.2 |
| Example 9 | | | 1.0 | 200 | 220 | 86.3/-1.4/7.2 | 8 |
| Example 10 | | | 1.2 | 200 | 220 | 84.3/0/8.5 | 10.3 |
| Comparative Example 1 | Sb=0.1 1 | 0.4 | | 180 | 210 | 82.3/-0.9/4.1 | 4.9 |
| | Sn=0.1 1 | | | | | | |
| Comparative Example 2 | Sb=0.0 3 | 0.2 | | 180 | 210 | 83.9/-1.3/4.1 | 7.9 |

| | | | | | | | |
|---|---|---|---|---|---|---|---|
| Sb: antimony tri-oxide Sn: dibutyl tin oxide Ti: tetra-n-butyl titanate (titanium content: 14.1%) Vertec VEXP 0641 (Johnson Matthey) (titanium content: 7.0%) Definition of the color indexes: L, a and b values L value means a whitening value in which a color is close to white as the value is closer to 100 a value means a green and red value in which a brighter color is shown as the value is closer to 0, wherein + stands for red and - means green. b value means a yellow and blue value, wherein + stands for yellow and - means blue. | | | | | | | |

As such, the color is shown to be brighter as the L value increases, and the tones of color are superior as the a and b values are getting closer to 0. Especially, an increase in b value indicates an increase in side-reactions.

In Examples 1 to 10, the resin was prepared by using titanium catalyst alone, in Comparative Example 1, additional catalysts, Sb and Sn, were used with the catalyst used in each of Examples 1 to 5, and in Comparative Example 2, an additional catalyst, Sb, was used with the catalyst used in each of Examples 6 to 10.

Accordingly, when comparing Examples 1 to 5 with Comparative Example 1, and when comparing Examples 6 to 10 with Comparative Example 2, a whitening value was shown to be higher when the reaction temperature was increased and the titanium catalyst was used alone, compared with case in which the heavy-metal catalyst such as Sb and Sn was used together. In addition, it was confirmed that the physical properties such as color indexes and MFI were attained at desired levels.

In particular, when the amount of titanium catalyst used was controlled as in Examples 2, 3 and 7, 8, the high L value was obtained compared with case in which the heavy-metal catalyst was used together, while maintaining the color indexes and MFI value at levels substantially similar to those of Comparative Examples.

As such, it was revealed that, by controlling an amount of a catalyst and the reaction temperature when a polyester resin is manufactured using an organic titanium catalyst, the resin possessing the physical properties similar to or more improved than the use of a conventional heavy-metal catalyst can be obtained.

It will be apparent to those skilled in the art that various changes and modifications can be made to the above-described exemplary embodiments of the present invention without departing from the scope of the invention. Thus, it is intended that the present invention covers all such modifications provided they come within the scope of the appended claims and equivalents thereof.

## Claims

1. A method of manufacturing a polyester resin comprising:
esterifying an alcohol monomer and a carboxylic acid monomer at a temperature of 200 to 220 °C in the presence of 0.1 to 1.5 g of an organic titanium catalyst with respect to 1 mol of the carboxylic acid monomer, and
polycondensing the esterification reaction product at a temperature of 220 to 240 °C under a vacuum of less than 2 Torr.

2. The method of claim 1, wherein, in the esterification of the alcohol monomer and the carboxylic acid monomer, the alcohol monomer is at least one selected from the group consisting of 1,4-butylene glycol, 1,3-butylene glycol, 1,3-propylene glycol and 1,2-ethylene glycol, and the carboxylic acid monomer is at least one selected from the group consisting of succinic acid, adipic acid, suberic acid, sebacic acid, terephthalic acid, their anhydrides and their derivatives.

3. The method of claim 1, wherein the organic titanium catalyst comprising 5 to 15 wt% of titanium is used in the esterification.

4. The method of claim 1, wherein the esterification is performed by further adding triphenyl phosphate or trimethyl phosphate.

5. A polyester resin manufactured by the method defined in any one of claims 1 to 4.

6. An article comprising the polyester resin defined in claim 5.

7. The article of claim 6, which is a diaper, a food packaging material or a medicine packaging material.

8. An organic titanium catalyst comprising 5 to 15 wt% of a titanium component used for polymerization a polyester resin.
